# EUROPEAN PATENT SPECIFICATION

(11) **EP 2 691 073 B1**
(45) Date of publication and mention of the grant of the patent: **31.05.2017**
(21) Application number: 12710725.8
(22) Date of filing: 26.03.2012
(51) Int. Cl.: A61K 8/34, A61K 8/36, A61K 8/368, A61K 8/66, A61Q 19/08, A61K 8/97, A61K 8/49

(54) **MODULATING EPIGENETIC DNA METHYLATION TO CAUSE CELLS TO ADOPT DNA METHYLATION PATTERNS ASSOCIATED WITH YOUNG CELLS**
MODULIERUNG DER EPIGENETISCHEN METHYLIERUNG SO DASS ZELLEN DNA METHYLIERUNGSMUSTER VON JUNGEN ZELLEN ANNEHMEN
MODULATION DE LA MÉTHYLATION ÉPIGÉNÉTIQUE DE L'ADN POUR ENTRAÎNER LES CELLULES À ADOPTER DES MOTIFS DE MÉTHYLATION DE L'ADN ASSOCIÉS À DES CELLULES JEUNES

(30) Priority: 27.03.2011 US 201161468053 P
(43) Date of publication of application: 05.02.2014
(73) Proprietor: Arch Chemicals, Inc., Allendale, New Jersey 07401-1629 (US)
(72) Inventor: GRUBER, James Vincent, Washington New Jersey 07882-4002 (US); LUDWIG, Philip Ledette, Dunellen New Jersey 08812-1250 (US)
(74) Representative: Riegler, Norbert Hermann
(86) International application number: PCT/EP2012/055286
(87) International publication number: WO 2012/130783

(56) References cited:
- EP-A2- 0 797 984
- WO-A1-01/43711
- JP-A- 2007 131 578
- DATABASE GNPD [Online] MINTEL; September 2009 (2009-09), "Instant Aqua Filler", XP002714171, Database accession no. 1184670 -& ISP: "Personal Care Reference Guide", , April 2009 (2009-04), pages 1-64, XP002714172, Retrieved from the Internet: URL:http://www.anshulindia.com/pdfs/ISP%20 Personal%20Care%20Reference%20Guide.pdf [retrieved on 2013-10-02]

## Description

### FIELD OF THE INVENTION

The present invention relates to compositions effective in controlling DNA methylation in human skin cells. More particularly, the present invention relates to topical compositions containing a plant meristem conditioned nutrient medium derived from the plants of the family *Poaceae,* and to the manufacture and the method of using such compositions.

### BACKGROUND OF THE INVENTION

Plants are unique among living organisms on the earth for being able to grow a new plant from a single cell taken from an older plant. The process of growing such plant cells are referred to as plant tissue culturing and has been known since the early 1900's.

Plant cell cultures have been used in topical applications. For example US 2008/0299092 A1 discloses the use of dedifferentiated plant cells derived from apples in cosmetic preparations for the purpose of influencing skin stem cell health. Likewise, EP 1 736 167 A2 discloses the topical application of plant cell culture media from *Syringa vulgaris* enriched in verboscosides intended to provide antioxidant influences to the skin. US 2007/0015252 A1 discloses the topical application of plant cultures from *Ajuga reptans* that contain phenylpropanoids to give the extract a high level of antioxidants. US Patent No. 6,551,625 discloses that undifferentiated plant cells grown as a suspension culture from *Salvia miltiorrhiza* can be used in cosmetic and topical applications to help prevent body odors from forming. EP 2 133 323 A1 discloses growing *Centella asiatica* as a tissue culture for production of 3,5-dicaffeoyl-4-malonylquinic acid that can have metalloproteinase inhibitory activity when used in cosmetic products for the skin. EP 0 797 984 A2 discloses anti-ageing cosmetics with an enzymolysed solution of alkali extract of rice (grain) extract. It is suitable in skin anti-ageing treatment and anti-wrinkle treatment, and promotes collagen production. However, heretofore, to the knowledge of the applicants, there is no prior art disclosing any topical treatments made from plant cell cultures intended to influence epigenetic DNA or chromatin methylation in skin cells.

Epigenetics is the study of heritable changes in gene expression that is caused by a mechanism other than changes to the DNA sequence. Any modification that alters gene activity without changing the DNA sequence and that can also be transmitted to daughter cells would be considered an epigenetic modification. Epigenetic changes are passed onto daughter cells and may last for multiple generations even though there is no change to the DNA sequence. Epigenetic factors and changes have been shown to play an important part in cellular differentiation, development, aging and disease (Cropley *et al*., 2006; Weaver *et al*., 2006; Rakyan *et al*., 2003).

DNA methylation at both the global and gene specific level has been shown to play an important role in regards to epigenetic memory; changes in DNA methylation have been shown to be age-related (Gopisetty *et al*., 2006; Ottaviano *et al*., 1994; Feng *et al*., 2006; Boks *et al*., 2009). DNA methylation in regards to epigenetic changes occurs at the cytosine-5 in CpG dinucleotides. The methyl group is attached to a cytosine base followed by a guanine dinucleotide base. The p represents the phosphate that links the C and G together. This helps differentiate CpG from a CG base pair. DNA methylation can also occur at CpA, CpT, and CpC sites, although currently the majority of the data linking cytosine methylation and gene transcription is from CpG data due to both biological and technical reasons.

Methylation of the CpG-rich promoter areas of genes, called CpG islands, has been identified as an essential mechanism in the regulation of gene transcription (Metivier et al., 2008; Bird, 2002). CpG islands have been defined by Takai and Jones (2002) as a region of 200 base pairs with a GC content of >55%. CpG islands are in approximately 70% of human promoters (Saxonov *et al*., 2006). Gene promoters are regions of the DNA upstream of a gene where RNA polymerase initially binds to begin transcription of the gene. Besides being associated with promoters, CpG islands are also linked to housekeeping genes. CpG islands are typically unmethylated in normal cells, thus allowing the transcriptional machinery access to the DNA. In certain diseases and with aging, CpG islands become aberrantly methylated, dis-regulating the usual transcriptional activity of the gene. The methylation occurs symmetrically on the cytosine residues on both strands of the CpG dinucleotides. DNA methylation is established early in an organism during embryogenesis. About 70-80% of CpG sites are methylated in mammalian cells but the CpG sites and their degrees of methylation are unevenly distributed in the genome (Bird *et al.,* 1985). CpG sites are primarily found within the promoters of ∼70 of human genes (Saxonov *et al*., 2006) and these sites tend to be unmethylated. When CpG islands in a promoter are methylated, this results in transcriptional repression of the adjacent gene. Expressed genes generally appear to have low methylation in their promoter region and high methylation in their gene body (Ball *et al*., 2009). DNA methylation is presumed to change the chromatin density and accessibility of the DNA to cellular machinery, thus influencing the transcriptional potential of the underlying DNA sequence.

DNA methylation levels within a specific cell at specific genes are subject to change over time. As are the DNA methylation levels when comparing different cell types such as human pluripotent stem cells and somatic cells (Deng *et al*., 2009). Monozygotic (identical) twins have the same genotype since they are derived from the same zygote. Studies have shown that patterns of epigenetic modification diverge in monozygotic twins as they grow older, which suggests that small defects in transmitting epigenetic information through successive cell divisions may occur (Fraga *et al*., 2005). This process, called "epigenetic drift", is associated with aging. Studies of twins have estimated that genetic factors only determine 20-30% of the variation in human lifespan. The remaining 70-80% of the variation is due to the environment and other non-genetic factors (Fraga *et al*., 2005; Mitchell *et al*., 2001; Herskind *et al*., 1996; Poulsen *et al*., 2007).

Methods to influence DNA methylation are known. For example, US Patent No. 7,790,746 discloses methods to inhibit DNA methylation using various quinoline derivatives. However, this patent fails to disclose the use of plant derived actives as a means to influence DNA methylation, particularly at the promoter of the gene.

What is presently needed in the cosmetic community are ingredients that improve the characteristics of aging skin by influencing the methylation of the promoter regions of the various critical genes associated with aging in mammalian, especially human, skin cells. The present invention provides an answer to that need.

### SUMMARY OF THE INVENTION

In one aspect, the present invention relates to a topical composition for controlling DNA methylation in human skin cells comprising: (a) a plant meristem conditioned nutrient medium which is a plant meristem cell suspension extract derived from the plants of the genus *Oryza*, (b) a preservative present at an amount sufficient to provide sterilizing or biostatic efficacy with respect to component (a); and (c) a dermatologically acceptable vehicle.

In another aspect, the present invention relates to a composition concentrate containing (a) a plant meristem conditioned nutrient medium (plant meristem cell suspension extract) derived from the plants of the genus *Oryza*, and (b) a preservative present at an amount sufficient to provide sterilizing or biostatic efficacy with respect to component (a), wherein component (a) is present in an amount of 0.0000001% to 99% by weight of the composition concentrate, with 90% to 99% being preferred, and 97% to 99% being more preferred, and component (b) is present in an amount of 0.01% to 10% by weight of the composition concentrate, with 0.1% to 5% being more preferred and 0.4% to 2% being most preferred.

In yet another aspect, the present invention relates to a process for preparing the topical composition described above. The process includes the steps of: (i) providing totipotent, undifferentiated plant cells derived from the plants of the genus *Oryza*; (ii) growing the plant cells in a liquid nutrient medium either in the presence of or the absence of chemical, gaseous, or energetic stresses intended to elicit expression of secondary plant cell metabolites to produce a mixture containing water-soluble and water-insoluble components, (iii) removing the water insoluble components to produce the plant meristem conditioned nutrient medium (plant meristem cell suspension extract), and (iv) combining the plant meristem conditioned nutrient medium (plant meristem cell suspension extract) with a preservative and a dermatologically acceptable vehicle, thereby making the topical composition.

In yet another aspect, the present invention relates to a method for controlling DNA methylation in human skin cells. The method includes contacting the skin with the topical composition of the invention.

### BRIEF DESCRIPTION OF FIGURES

Fig. 1 is a HPLC chromatogram of an ozone stressed meristem conditioned nutrient medium (plant meristem cell suspension extract).
Fig. 2 is a HPLC chromatogram of a non-ozone stressed rice meristem conditioned nutrient medium (plant meristem cell suspension extract).
Fig. 3 is a graph illustrating the effect of a plant meristem conditioned nutrient medium (plant meristem cell suspension extract) of the invention on the level of methylation at gene promoters genome wide of *in vitro* grown fibroblasts.
Fig. 4 is a graph illustrating the effect of a plant meristem conditioned nutrient medium (plant meristem cell suspension extract) of the invention on the level of methylation at collagen 1A1 gene promoters of *in vitro* grown fibroblasts.
Fig. 5 is a graph illustrating the effect of a plant meristem conditioned nutrient medium (plant meristem cell suspension extract) of the invention on the level of methylation at collagen 1A2 gene promoter of *in vitro* grown fibroblasts.
Fig. 6 is a graph illustrating the effect of a plant meristem conditioned nutrient medium (plant meristem cell suspension extract) of the invention on the collagen 1A protein levels of *in vitro* grown fibroblasts.
Fig. 7 is a graph illustrating the effect of a plant meristem conditioned nutrient medium (plant meristem cell suspension extract) of the invention on the dermatopontin protein expression of *in vitro* grown fibroblasts.

### DETAILED DESCRIPTION OF THE INVENTION

It has now been surprisingly found that plant meristem conditioned nutrient media which are meristem cell suspension extracts, derived from the plants of the genus *Oryza*, more preferably species *sativa*, when applied topically to human skin cells such as, for example, fibroblasts, keratinocytes, melanocytes, and the like, can modulate DNA methylation in young and more particularly intrinsically and extrinsically aged mammalian skin cells, especially at the promoter region of the genes. The treated cells show DNA methylation patterns more characteristic of those found in young, un-aged cells. The topical compositions containing these plant meristem conditioned nutrient media are particularly advantageous for use in human and veterinary therapy and for nutritional and cosmetic purposes.

Accordingly, in one embodiment, the present invention provides a topical composition for controlling DNA methylation in human skin cells comprising: (a) a plant meristem conditioned nutrient medium (plant meristem cell suspension extract) derived from the plants of the genus *Oryza*, (b) a preservative present at an amount sufficient to provide sterilizing or biostatic efficacy with respect to component (a); and (c) a dermatologically acceptable vehicle.

In connection with the topical composition of the invention described above, component (a) is present in an amount of 0.0000001% to 99% by weight of the composition, with 0.0001% to 50% being preferred, and 0.001% to 25% being more preferred and 0.01% to 10% being most preferred, component (b) is present in an amount of 0.01% to 10% by weight of the composition, with 0.1% to 5% being more preferred and 0.4% to 2% being most preferred, and component (c) is present in an amount of 1% to 98% by weight of the composition, with 80% to 90% being most preferred.

Component (a), a plant meristem conditioned nutrient medium (plant meristem cell suspension extract) suitable for use in the topical composition of the invention is derived from the plants of the genus *Oryza*, preferably from plants *Oryza sativa.*

The plant meristem conditioned nutrient media (plant meristem cell suspension extracts) can be prepared by a process including the steps of (i) growing plant meristem cultures derived from the plants of the genus *Oryza* in a liquid nutrient medium to produce a mixture containing water-soluble and water-insoluble components, and (ii) removing the water insoluble components to produce the plant meristem conditioned nutrient media (plant meristem cell suspension extracts).

The method to create plant meristem cultures are known in the art. Typically, the plants are first sterilized, then the meristem is excised from the plant and placed on a solid nutrient medium in order for the undifferentiated callus tissues to form from the meristem.

Solid nutrient media are generally known to a person skilled in the art. In one embodiment, they contain factors such as Murashige & Skoog medium, sucrose, Gamborg vitamins, growth hormones such as IAA, kinetin, and agar.

For the purposes of the present invention, it is preferable to maintain the callus tissues formed form the meristem as undifferentiated cells. In the nutrient media, a relatively high level of auxin to kinetin favors rooting, while the reverse can lead to shoot formation and intermediate levels to the proliferation of callus. Accordingly, the auxin and kinetin levels in the nutrient media should be maintained so that only undifferentiated cells are produced.

After the meristem grows in the solid media for a suitable period of time, the callus tissues are sub-cultured by dividing and transferring onto new solid media. Preferably, this process is repeated at least several times in order to obtain stable cell lines. As used herein, stable cell lines means the cells that have constant traits, produce a reproducible amount of actives, grow at a constant rate, and look the same after multiple generations. Typically, stabilized cultures have little variation within the culture and through time and replication will have little variation.

As those skilled in the art know, tissue cultures can produce clonal variants. Within a callus or plant tissue, there is natural variation within the cells. This somaclonal variation is dependent on the variation in a population of cells, either pre-existing or culture-induced. The variation may be genetic or epigenetic. These changes in the callus or suspension cultures have a significant benefit in the production of secondary metabolic actives from the suspension cultures. Therefore, for the purposes of the present invention, sub-culture cell lines are chosen that are fast growing, have stable traits such as color and size, grow at a constant rate and other qualities of interest.

After a cell line is determined to be stable, cells from the solid medium, i.e., totipotent, undifferentiated plant cells, are inoculated into a liquid medium. A liquid medium is known to a person skilled in the art. It contains factors such as Murashige & Skoog medium, sucrose, Gamborg vitamins, growth hormones such as IAA, and kinetin.

In one embodiment, the cells are allowed to grow in a liquid nutrient medium in the presence of chemical, gaseous, or energetic stresses intended to elicit expression of secondary plant cell metabolites.

Although a variety of chemical or energy stresses may be used to induce secondary metabolite production, ozone is a preferred stressor as it is easily introduced into the cell cultures through the oxygen feed used to keep the cells in an oxygenated environment in the reactor.

Exposure to ozone helps push the cells to synthesize more secondary metabolites. Secondary metabolites of the present invention include, but are not limited to, plant growth regulators, plant cytokines, flavonoids, carotenoids, phytosterols, saponins, glucosinolates, protease-inhibitors, phytoestrogens, sulfides, phytic acids, alkaloids, terpenoids, glycosides, phenols, phenazines, polyketides, peptides, polyphenols and such.

Under a certain threshold, a mild stress from ozone may be compensated by the plant causing it to produce more actives. Whereas, at higher levels, the detrimental effects of the severe stress may cause irreversible damages. Furthermore, the stress tolerance threshold depends not only on the type of stressor, in the case of ozone, and exposure time, but also on the species specific plant stress-coping capacity. It is possible to optimize the amount of ozone applied to the rice suspension culture to achieve specific stress-related results. Typical levels of ozone useful for the present invention can be in the range of from 0.0001 to 50 mM. More preferred are ozone levels of from 0.01 mM to 5 mM. Most preferred are ozone levels from 0.1 mM to 0.5 mM. The plant cells may be exposed to the ozone for several minutes to several days depending on species of plant, ozone concentration, aeration rates, temperature, and the like.

Another way to monitor oxidative stress in the plant meristem conditioned nutrient media fermentations is to apply the ozone until a certain number of plant cells have died. Methods to measure plant cell viability are well known to those skilled in the art and can include, for example, the MTT assay which is a colorimetric assay that demonstrates viable mitochondrial functions thus demonstrating cells that are living from those that are dead. A preferred cell viability count would be 50%, a more preferred level would be 75%, and a most preferred level would be 80% viability of the plant cells after application of the ozone stress.

Commercially available ozone generators used for the ozone treatment of the plant cells can be found at, for example, Erwin Sander Elektroapparatebau GmbH, Uetze-Eltze, Germany. In most circumstances, an ozone detector is used in addition to the ozone generator. Suitable ozone detectors are available from Dasibi, Glandlae, California.

Although ozone is described here as a preferred stressor, other stressors known to those skilled in the art can also be used to induce secondary metabolites. These stressors include, but are not limited to, plant growth regulators, plant pathogen proteins and polymers such as chitin and various forms of external energy including but not limited to heat and UV radiation.

The cells are allowed to grow for a certain amount of time until a sufficient quantity of biomass is obtained. If necessary, fresh liquid nutrient medium can be added. In one embodiment, the cells are allowed to grow to an optical density of 100-400, after which, the biomass is harvested and an extraction is performed to provide the plant meristem conditioned nutrient medium. The extraction process is known to a person skilled in the art, for example, one can filter off the insoluble components and the obtained soluble components as the plant meristem conditioned nutrient medium (plant meristem cell suspension extract).

Without being bound by any theory, it is believed that through the plant cell growth, the cells are able to condition the nutrient media by metabolizing compounds in the media and excreting some of these metabolites along with various other plant cell produced compounds into the media. Since the initial source of plant tissue is from the plant meristem, for the purposes of instant patent application, the conditioned media are called "plant meristem conditioned nutrient media".

If necessary, the thus obtained plant meristem conditioned nutrient media (plant meristem cell suspension extracts) can be further processed by methods known to those skilled in the art to improve color or odor or to concentrate or even dry the product completely.

The efficacy of the plant meristem conditioned nutrient media (plant meristem cell suspension extracts) may be further enhanced by including the media in delivery systems such as, for example, liposomes, niasomes, polymerisomes, dendrimerosomes and the like or through penetration enhancing mechanisms such as, for example, iontophoresis.

The plant meristem conditioned nutrient media (plant meristem cell suspension extracts) may also be included in anhydrous systems by removal of the extraction solvent by any method known to those skilled in the art such as, for example, freeze drying, spray drying, belt drying and the like. In the anhydrous form, the media can be included in anhydrous gels, such as, for example, silicone gels or in anhydrous powders such as, for example, foundations and pressed powders.

The plant meristem conditioned nutrient media (plant meristem cell suspension extracts) are suitable for use in the topical composition of the invention. In addition, this conditioned media may also be the basis for further modifications though additional plant or microorganism fermentation where the plant meristem conditioned nutrient medium is added to an ongoing fermentation process as a secondary source of fermentation nutrients.

Component (b) of the topical composition of the present invention is a preservative present at an amount sufficient to provide sterilizing or biostatic efficacy with respect to component (a), the plant meristem conditioned nutrient medium.

Preservatives are well known to those skilled in the art and can include, for example, ingredients such as organic acids, such as for example, salicylic acid or sorbic acid, organic alcohols such as, for example, phenoxyethanol, benzyl alcohol, glycols and ethanol, quaternary ingredients such as, for example, Quaternium-15, enzymes such as, for example, glucose oxidase and lactoperoxidase in combination with a substrate such as glucose sold commercially as Biovert by Arch Personal Care (South Plainfield, NJ) and the like or combinations of these preservatives.

Preferred preservatives are selected from the group consisting of salicylic acid, sorbic acid, phenoxyethanol, benzyl alcohol, ethanol, Quaternium-15, glucose oxidase and lactoperoxidase in combination with a substrate, and combinations thereof.

The preservative should be added to the plant meristem conditioned nutrient media (plant meristem cell suspension extracts) at a concentration sufficient to either act as an antibiotic, that is essentially sterilizing the plant meristem conditioned nutrient media, or as a biostatic agent, maintaining the plant meristem conditioned nutrient media in a state such that living microorganisms present in the media do not reproduce. Methods for determining the efficacy of a preservative system are well known to those skilled in the art. The most popular method for testing preservative efficacy is called challenge testing. In this test method, microorganisms are introduced into the liquid plant meristem conditioned nutrient media in a controlled fashion and allowed to try and grow on the media. If the microorganism levels either diminish or remain unchanged over a prescribed time, the medium is said to be adequately protected from microbial contamination.

A particularly preferred preservative is phenoxyethanol.

The preservative, and in particular phenoxyethanol, is preferably used at a level of at least 0.5 wt%, more preferably 1.0 wt%, most preferred 1.2 wt% of the composition.

If the product is isolated as a solid, a preservative will likely still be required to maintain the product long enough to allow for drying and will remain in the powdered product after the drying event. Methods of drying are well known to those skilled in the art and can include, for example, spray drying, freeze drying, drum drying, film drying and the like. An especially preferred method of drying is spray drying methods of which are disclosed, for example, in US 2003/0198682 A1.

Components (a) and (b) have been described above. Component (c) of the composition of the invention is a dermatologically acceptable vehicle. The phrase "dermatologically acceptable vehicle", as used herein, means that the vehicle is suitable for topical application to the skin, has good aesthetic properties, is compatible with the actives of the present invention and any other components, and will not cause any untoward safety or toxicity concerns.

The vehicle can be in a wide variety of forms. For example, emulsion carriers, including, but not limited to, oil-in-water, water-in-oil, water-in-oil-in-water, and oil-in-water-in-silicone emulsions, are useful herein. These emulsions can cover a broad range of viscosities, e.g, from about 100 cps to about 200,000 cps. These emulsions can also be delivered in the form of sprays using either mechanical pump containers or pressurized aerosol containers using conventional propellants. These carriers can also be delivered in the form of a mousse. Other suitable topical carriers include anhydrous liquid solvents such as oils, alcohols, and silicones (e.g., mineral oil, ethanol, isopropanol, dimethicone, cyclomethicone, and the like); aqueous-based single phase liquid solvents (e.g., aqueous-alcoholic solvent systems); and thickened versions of these anhydrous and aqueous-based single phase solvents (e.g., where the viscosity of the solvent has been increased to form a solid or semi-solid by the addition of appropriate gums, resins, waxes, polymers, salts, and the like). Examples of topical carrier systems useful in the present invention are described in the following four references: "Sun Products Formulary" Cosmetics & Toiletries, vol. 105, pp. 122-139 (December 1990); "Sun Products Formulary", Cosmetics & Toiletries, vol. 102, pp. 117-136 (March 1987); U.S. Pat. No. 4,960,764 to Figueroa et al.; and U.S. Pat. No. 4,254,105 to Fukuda et al.

A more detailed discussion of suitable vehicles is found in U.S. Pat. No. 5,605,894 issued to Blank et al., and, U.S. Pat. No. 5,681,852 to Bissett. The vehicle can also be a powdered-type topical composition such as, for example, a powdered foundation.

Additionally, the topical composition of the present invention can optionally contain other functional ingredients such as, water, surfactants, emulsifiers, conditioners, emollients, waxes, oils, polymers, thickeners, fixatives, colorants, humectants, moisturizers, stabilizers, diluents, solvents, fragrances and the like, as well as active ingredients such as, for example, botanicals, neutraceuticals, cosmeceuticals, therapeutics, pharmaceutics, antifungals, antimicrobials, steroidal hormones, antidandruff agents, anti-acne components, sunscreens, preservatives and the like.

The topical composition of the invention may be in any form known to a person skilled in the art, for example, a cream, lotion, gel, serum, soap, stick, powder or the like. The composition can be applied as a leave-on product or as a product intended to be rinsed off the skin immediately after applications such products including things like body washes, shampoos and the like.

It has been surprisingly found that application of the topical compositions of the present invention can influence age-related epigenetic alterations and delay and alleviate certain aspects of aging of the skin. Through the topical application of compositions containing plant meristem conditioned nutrient media (plant meristem cell suspension extracts), it has been surprisingly found that modulation of DNA methylation can occur in such a way that aged cells undergo a transformation and gain back the DNA CpG methylation patterns that their younger, unaged cells had. DNA methylation patterns at both the genomic level and those at specific genes can be modulated through application of the plant meristem conditioned nutrient media. The identification that both the genome and individual genes have reversible methylation states and that the plant meristem conditioned nutrient media can influence the methylation state of skin cells, results in new topical therapeutic opportunities.

Although the genome may be exactly the same in two individuals, regulating gene expression through epigenetic modifications can play a role in vast differences between longevity and fitness of the organism or tissue. Through modulating the cellular DNA methylation patterns, opportunities to potentially treat hair loss, skin inflammatory states, pigmentation and age spots, wrinkles, drying of the skin related to aging, and loss of elasticity and skin texture, among other ailments, can be achieved.

Epigenetic patterning and maintenance are of high importance for normal cellular functioning. By profiling the CpG methylation from young and aged skin tissues, characterization of the role of aging and UVB exposure in regards to methylation variation can be noted. Because the strongest positive correlation between aging and methylation occurs at loci in CpG islands in the promoter regions of the gene, studies on the efficacy of the plant meristem conditioned nutrient media focused on these important sites of methylation.

In another embodiment, the present invention also relates to a composition concentrate comprising: (a) a plant meristem conditioned nutrient medium which is a plant meristem cell suspension extract derived from the plants of the genus *Oryza*, (b) a preservative present in an amount sufficient to provide sterilizing and biostatic efficacy with respect to component (a), wherein component (a) is present in an amount of 0.0000001% to 99% by weight of the composition, with 90% to 99% being preferred, and 97% to 99% being more preferred, and component (b) is present in an amount of 0.01% to 10% by weight of the composition, with 0.1% to 5% being more preferred and 0.4% to 2% being most preferred.

In a preferred embodiment component (a) of the composition concentrate is a plant meristem conditioned nutrient medium which is a plant meristem cell suspension extract derived from the plants of *Oryza sativa.*

The composition concentrate of the present invention can be suitable incorporated into a topical composition as described above by any methods known to a person skilled in the art.

Yet another embodiment of the invention is a process for preparing the composition of the invention, said process comprising the steps of
(i) providing totipotent, undifferentiated plant cells derived from the plants of the genus *Oryza*;
(ii) growing the plant cells in a liquid nutrient medium either in the presence of or the absence of chemical, gaseous, or energetic stresses intended to elicit expression of secondary plant cell metabolites to produce a mixture containing water-soluble and water-insoluble components,
(iii) removing the water insoluble components to produce the plant meristem conditioned nutrient medium which is a plant meristem cell suspension extract, and
(iv) combining the plant meristem conditioned nutrient medium with a preservative and a dermatologically acceptable vehicle, thereby making the composition of the invention.

In a preferred embodiment of the process the plant cells of step (i) are derived from plants of *Oryza sativa.*

In another preferred embodiment of the process the plant cells grow in the presence of ozone at step (ii).

In a particularly preferred embodiment of the process the plant cells of step (i) are prepared by a method comprising the steps of
(1) growing plant meristems derived from the plants of the genus *Oryza* in a solid nutrient medium; and
(2) selecting stabilized cultures, thereby producing said plant cells.

Yet another embodiment of the invention is a method for controlling DNA methylation in human skin cells comprising contacting the skin with a topical composition comprising:
(a) a plant meristem conditioned nutrient media derived from the plants of the genus *Oryza*,
(b) a preservative present in an amount sufficient to provide sterilizing or biostatic efficacy with respect to component (a); and
(c) a dermatologically acceptable vehicle.

The above method for controlling DNA methylation in human skin cells is preferably non-therapeutic, e.g. cosmetic.

The invention is further described in the Examples given below. The following examples are intended to illustrate the scope of the present invention. All percentages given herein are weight percents based on the total weight of the composition, unless otherwise stated.

### Example 1:

### Production of a plant meristem conditioned nutrient medium from Oryza sativa (rice)

Rice meristem cultures were grown on a solid medium (Murashigie & Skoog with Gamborg 1X vitamins, 30 g/L sucrose, 1 mg/L kinetin, 0.2 mg/L IAA, and 1% agar at pH 5.6) and subcultured for at least three months. Callus from the same culture line was inoculated into multiple 250 mL flasks containing approximately 60 mL of the above medium without agar. After two weeks, the culture was moved into a 500 mL flask that contained approximately 130 mL of liquid medium, which contained Murashige & Skoog medium, Gamborg 1X vitamins, 30 g/L sucrose, 1 mg/L kinetin, 0.2 mg/L IAA, at pH 5.6. After two weeks, the culture was moved into a 1 L flask that contained approximately 300 mL of liquid medium. Following fermentation for 14 days, 600 mL of culture was moved into a 3 L bioreactor with a working volume of 2.3 L. After three weeks of fermentation, 5 L of culture was moved into a 30 L bioreactor with a working volume of 22 L. Following fermentation for three weeks, 40 L of culture was moved into a 250 L bioreactor with a working volume of 200 L. After four weeks of fermentation, 200 L was moved into a 1000 L bioreactor with a total of 400 L of liquid medium. Following 2 weeks of fermentation, 350 L of fresh medium was added to bring to total volume to 750 L. The culture was allowed to grow for 2 weeks after which 0.5 ppm of ozone was added to the air line. The culture grew for one additional week to produce secondary metabolites, after which the culture was homogenized using a Niro homogenizer and filtered to remove solid material. The resulting material was referred to as rice meristem conditioned nutrient medium (plant meristem cell suspension extract) or as rice culture for short.

### Example 2:

### HPLC chromatogram of ozone stressed rice meristem conditioned nutrient medium of

### Example 1

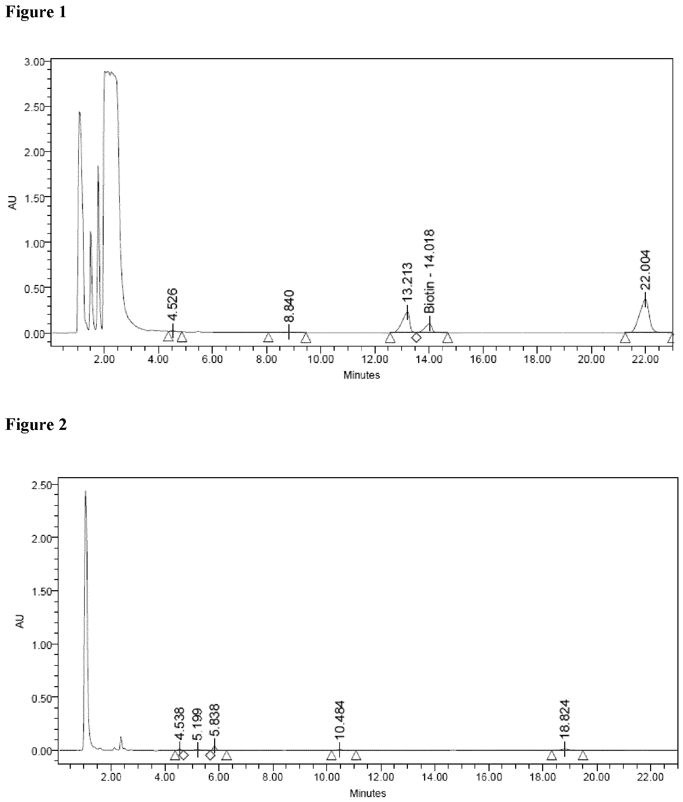

HPLC analysis was performed on the meristem conditioned nutrient medium extract of Example 1. Multiple peaks were observed but only one was from biotin denoting the ozone causes the production of secondary metabolites by the rice cultures. Analysis was performed using a Phenomenex Kinetex 2.6 µm, 100×4.6 mm column. Solvent A was methanol. Solvent B was 50 mM sodium phosphate (pH 4.5). Solvent A was used at 10% and solvent B was used at 90% isocratically. Flow was 1.0 mL per minute and the column temperature was 30 °C. Detection was done at 200 nm wavelength. The HPLC analysis result is shown in Fig. 1.

### Example 3:

### HPLC chromatogram of non-ozone stressed rice meristem conditioned nutrient medium

A rice meristem extract was prepared according to the procedure of Example 1 except without the addition of ozone. HPLC analysis of was performed under the conditions as described in Example 1. The result is shown in Fig. 2. As shown in Fig. 2, very few secondary metabolite peaks were observed in the HPLC chromatogram.

### Example 4:

### Mean CpG methylation at all gene promoters genome wide in in vitro grown fibroblasts decreases with application of ozonized rice meristem conditioned nutrient medium of

### Example 1

As described above, an important trait of young cells is that they have low levels of methylation compared to older cells. This example shows that application of 2% plant meristem conditioned nutrient medium of Example 1 applied *to in vitro* grown fibroblasts caused the cells to have a decreased level of methylation at gene promoters genome wide as compared to untreated control.

### Summary of Test Method

For this study human dermal fibroblasts were either taken through a period of combined intrinsic and extrinsic aging (sequential cell passages and repeated UVB exposure) or not aged. For the intrinsic aging procedure the fibroblasts were taken through 8 cell culture passages (3 weeks in culture). Cells were grown in 12 well plates and grown until confluent (every 48 to 72 hours) and harvested and reseeded at 50% of the original cell density so that each passage roughly represented one population doubling. For the extrinsic aging aspect, the cells were irradiated with UVB three times per week. While the original intention of the study was to take this group through 8 passages as well this was not possible. By the third passage the rate of cell replication had slowed to the point to where it was no longer possible to pass the cells. This group was kept in culture for 3 weeks and was subjected to 9 UVB exposures.

In addition to the aging treatments, the fibroblasts were also treated with test materials which were present during the entire aging process. The list of treatment groups is as follows:
1. Non-Aged Cells (harvested after a few days in culture)
2. Intrinsic Aging + Extrinsic Aging
3. Intrinsic Aging + Extrinsic Aging + 2% rice culture from Example 1
At the end of the culture period, the cell culture medium was collected and analyzed for changes.

### Methods

### Preparation and Treatment of Fibroblasts

Fibroblasts were seeded into the individual wells of a 12-well plate in 1 mL of Fibroblast Growth Medium (FGM) and incubated at 37±2 °C and 5±1% CO₂ with the media changed every 48 to 72 hours. Upon reaching confluency the cells were harvested and then reseeded at 50% of their original density. For the UVB exposures in the intrinsic plus extrinsic aging groups, the cell culture medium was replaced with phosphate buffered saline and the cells were exposed to 5-7 mJ/cm² of UVB. This exposure was applied three times a week with at least 24 hours between exposures.

The cells were cultured for 3 weeks as described above. During this time the intrinsic plus extrinsic aging group went through 3 population doublings and 9 UVB exposures. At the end of the three week period the cell culture medium and cells were collected 48 hours after the last medium change. The cells were rinsed with phosphate buffered saline to remove any residual test material and then 200 µL of Lysis Buffer (1 mM EDTA, 0.5% Triton X-100, 10 mM NaF, 150 mM NaCl, 20 mM β-glycerophosphate, 1 mM DTT, 10 µg/mL leupeptin, 10 µg/mL pepstatin, 3 µg/mL aprotinin prepared in phosphate-buffered saline) was added to each well. The cells were incubated for approximately 15 minutes on ice to allow for complete lysis. The lysates were then collected and stored at -75 °C until analyzed.

### Methylation array protocol

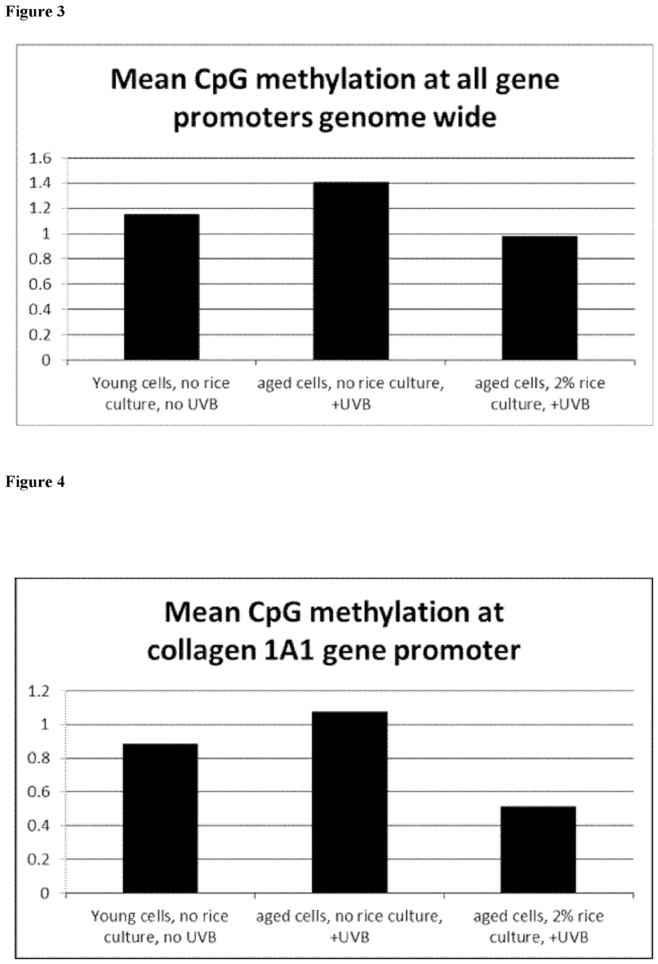

The DNA CpG methylation array chip is from Agilent and covers 27,627 CpG Islands. For the array, total genomic DNA (5 µg) was isolated from *in vitro* fibroblast cells, and then sheared into fragments (approximately 400 to 800 bases in length). From this 5 µg sample of sheared genomic DNA, 1 µg of total genomic DNA was set aside while the remaining 4 µg were used to isolate methylated DNA. The methylated DNA was separated using immuno-precipitation with an antibody specific for methylated DNA. The 1 µg of total genomic DNA and the methylated DNA were then fluorescently labeled and applied to the DNA methylation array. After hybridizing, the array was then scanned and the fluorescence intensity of the features on the array were then determined. The values for all CpG sites in all gene promoters were averaged to determine the mean. The results are reported in Fig. 3.

The data in Fig. 3 demonstrate that topical application of 2% of the rice meristem conditioned nutrient medium shows a reduction in global CpG methylation in the promoter region in fibroblasts intrinsically and extrinsically aged as compared to similar cells without treatment.

### Example 5:

### Mean CpG methylation at collagen 1A1 promoter in in vitro grown fibroblasts decreases with application of ozonized rice meristem conditioned nutrient medium of Example 1

One of the genes which has increased promoter methylation during aging is collagen 1A1 (Takatsu *et al*., 1999). This example illustrates that application of 2% rice meristem conditioned nutrient media from Example 1 *to in vitro* grown fibroblasts caused the cells to have a decreased level of methylation at the collagen 1A1 gene promoter as compared to similar cells without treatment. Assay was performed as described above. Methylation levels at all CpG sites in the collagen 1A1 promoter were averaged and the mean was found. The results are reported in Fig. 4.

The data in Fig. 4 demonstrates that topical application of 2% rice meristem conditioned nutrient medium from Example 1 demonstrates a reduction in DNA CpG methylation at the collagen 1A1 gene promoter in cells that have been intrinsically and extrinsically aged compared to similar cells without treatment.

### Example 6:

### Mean CpG methylation at collagen 1A2 promoter in in vitro grown fibroblasts decreases with application of ozonized rice meristem conditioned nutrient media of Example 1

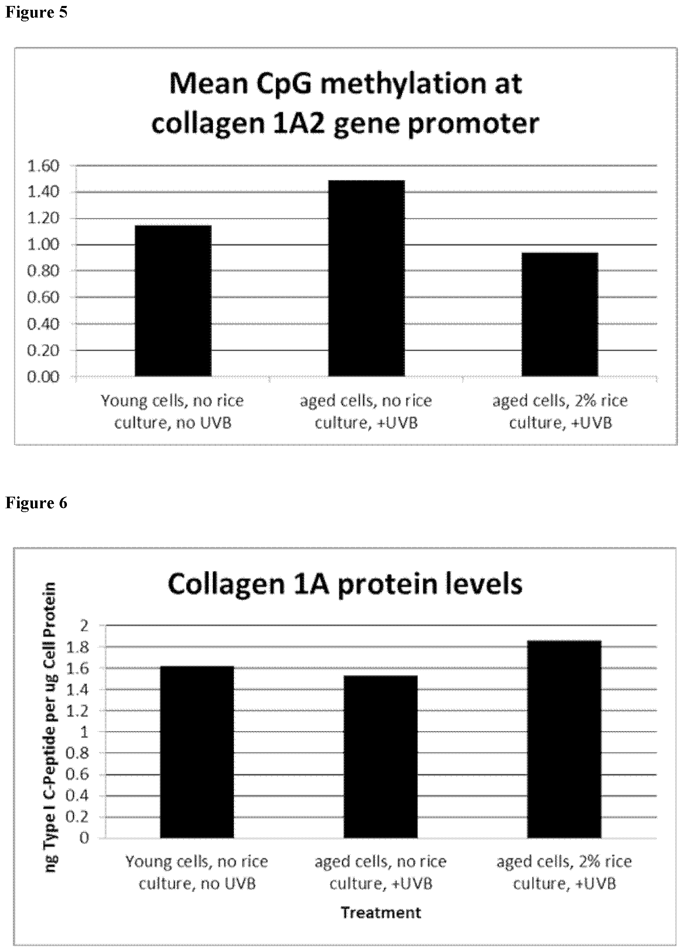

This example illustrates that application of 2% rice meristem conditioned nutrient medium *to in vitro* grown fibroblast cells caused the cells to have a decreased level of methylation at the collagen 1A2 gene promoter. Assay was performed as described above. Methylation levels at all CpG sites in the collagen 1A2 promoter were averaged and the mean was found. The results are reported in Fig. 5.

The data in Fig. 5 demonstrate that topical application of 2% rice meristem conditioned nutrient medium from Example 1 demonstrates a reduction in DNA CpG methylation at the collagen 1A2 gene promoter in cells that have been intrinsically and extrinsically aged compared to similar cells grown without treatment.

### Example 7:

### Collagen protein levels in in vitro grown fibroblasts increases with application of ozonized rice meristem conditioned nutrient medium of Example 1

This example illustrates that application of 2% rice meristem conditioned nutrient medium *to in vitro* fibroblast cells caused the cells to increase their production of collagen. Cells were grown and treated as above. The collagen assay was performed on both the media samples collected at 24 hours and the media collected at the 48 hour time point. For the assay, a series of type I C-peptide standards was prepared ranging from 0 ng/mL to 640 ng/mL. Next, an ELISA microplate was prepared by removing any unneeded strips from the plate frame followed by the addition of 100 µl of peroxidase-labeled anti procollagen type I-C peptide antibody to each well used in the assay. Twenty (20) µL of either sample (collected tissue culture medium) or standard was then added to appropriate wells and the microplate was covered and allowed to incubate for 3±0.25 hours at 37 °C. After the incubation the wells were aspirated and washed three times with 400 µL of wash buffer. After the last wash was removed 100 µL of peroxidase substrate solution (hydrogen peroxide + tetramethylbenzidine as a chromogen) was added to each well and the plate was incubated for 15±5 minutes at room temperature. After the incubation 100 µL of stop solution (1 N sulfuric acid) was added to each well and the plate was read using a microplate reader at 450 nm. The results are reported in Fig. 6.

The data in Fig. 6 demonstrate that topical application of 2% rice meristem conditioned nutrient medium from Example 1 demonstrates an increase in Type 1A collagen in cells that have been intrinsically and extrinsically aged compared to similarly cells grown without treatment.

### Example 8:

### Dermatopontin protein levels in in vitro grown fibroblasts increases with application of ozonized rice meristem conditioned nutrient medium from Example 1

This example illustrates that application of 2% rice meristem conditioned nutrient medium from Example 1 *to in vitro* grown fibroblast cells caused the cells to increase their production of dermatopontin. Cells were grown and treated as above. Dermatopontin protein levels were assayed using immunoblotting.

### Microfiltration Blotting of Cell Lysate and Immunodetection

A membrane was equilibrated in Tris Buffered Saline (TBS: 20 mM Tris, pH 7.5, 150 mM NaCl) and assembled into a Bio-Dot microfiltration apparatus. After assembly, 200 µL of TBS was added to each well used in the Bio-Dot and the vacuum was applied to ensure that there was adequate flow through all of the wells. Next, each cell lysate sample (approximately 5 µg) was assigned a well in the apparatus and was applied to the appropriate well. The samples were filtered under low vacuum. TBS was added to wells not assigned a sample to ensure that the membrane did not dry out during the procedure. At the end of the blotting procedure an additional 200 µL of TBS was applied and filtered through each well. The membrane was then removed from the Bio-Dot apparatus, washed in TBS for 5-10 minutes and then placed into blocking solution (Tris Buffered Saline [20 mM Tris, pH 7.5, 150 mM NaCl, 1% non-fat milk powder]) and allowed to incubate for at least 1 hour at room temperature on a rocking platform.

### Antibody Incubation and Detection

After blocking, the membrane was transferred to 20 mL of TBST (TBS with 0.1% Tween-20) and 0.1% non-fat powdered milk with an appropriate dilution of detection antibody and allowed to incubate overnight at 4 °C on a rocking platform. After this incubation the membrane was washed 3 times (1× for 15 minutes and 2× for 5 minutes) in TBST. The secondary antibody (conjugated with a fluorophore) was then incubated with the membrane in 15 mL of TBST with 0.1% non-fat powdered milk for 1 hour at room temperature and then washed 3 times with TBS (1× for 15 minutes, 2× for 5 minutes).

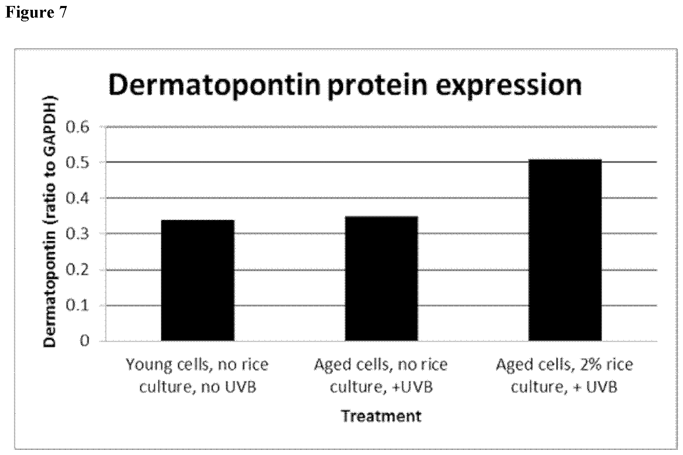

After the final wash, the membrane was placed into a BioRad Molecular Imager FX and scanned using an excitation laser and emission filter combination appropriate for the fluorophore. Images produced by the scanner were then analyzed using ImageJ image analysis software. The results are reported in Fig. 7.

The data in Fig. 7 demonstrate that topical application of 2% rice meristem conditioned nutrient medium from Example 1 caused a significant increase in expression of dermatopontin in aged cells verses untreated cells grown under similar conditions.

### Example 9:

### Oil-in-water emulsions

The rice meristem conditioned nutrient medium from Example 1 ("rice culture") was formulated into an oil-in-water emulsion using the following formulation and process:

**Oil in Water Emulsion containing Extract from Example 1**

| **Ingredient** | **INCI Nomenclature** | **%** |
|---|---|---|
| Water | Water | q.s |
| Versene 100 | Tetrasodium EDTA | 0.10 |
| Glycerin | Glycerin | 2.00 |
| Carbopol Ultrez 10 | Carbomer | 0.20 |
| Brookswax D | Cetearyl Alcohol & Ceteareth-20 | 2.00 |
| Liquiwax DIADD** | Dioctyldodecyl Dodecanedioate | 5.00 |
| Loronate TMP-TC | Trimethylolpropane Tricaprylate / Tricaprate | 2.00 |
| Arlacel 60 | Sorbitan Stearate | 1.50 |
| Stearyl Alcohol | Stearyl alcohol | 0.20 |
| Cetyl Alcohol | Cetyl Alcohol | 0.50 |
| Stearic Acid | Stearic Acid | 0.50 |
| Myritol 318 | Caprylic/Capric Triglyceride | 2.00 |
| DC 200/100 cst | Dimethicone | 0.75 |
| Water | Water | 5.00 |
| TEA 99 | Triethanolamine | 0.25 |
| Rice Culture from Example 1 | - | 1.00 |
| Mikrokill COS | Phenoxyethanol & Caprylyl Glycol & Chlorphenesin | 0.75 |

### Procedure:

Combine Phase A and heat to 75 °C. Mix until uniform.
Combine Phase B and heat to 75 °C Mix until uniform.
With slow mixing, add Phase B to Phase A. Mix for 20 minutes.
Add pre-mix Phase C and mix until uniform. Turn off the heat.
In side kettle pre-mix Phase D and add to the batch below 40 °C. Mix until uniform. Add Mikrokill COS and fragrance of Phase E, and mix until uniform.

### Example 10:

### Water-in-oil emulsions

The rice culture from Example 1 was formulated into water-in-oil emulsions using the following formulation and process:

**Water in Oil Emulsion containing Rice meristem conditioned nutrient media**

| **Ingredient** | **INCI Nomenclature** | **%** |
|---|---|---|
| Water | Water | q.s to 100 |
| Glycerin | Glycerin | 3.00 |
| Sodium Chloride | Sodium Chloride | 1.00 |
| Rice Culture from Example 1 | - | 1.00 |
| Mikrokill COS | Phenoxyethanol & Caprylyl Glycol & Chlorphenesin | 0.75 |
| SF1328 | Cyclomethicone & Dimethicone Copolyol | 10.00 |
| SF 1202 | Cyclomethicone | 8.50 |
| Gel Base Sil | Cyclomethicone & Dimethicone | 1.50 |
| Gel Base BSM-PE | Cyclomethicone & Dimethicone & Phenyl Trimethicone & Polyethylene | 1.50 |
| | | 100.00 |

### Procedure:

Mix all ingredients of Phase A together.
Combine Phase B ingredients in order shown, thoroughly mixing each component until homogeneous before adding the next ingredients.
Slowly add Phase A to Phase B with good mixing. Gradually increase agitation to high shear as mixture thickens. Continue agitation for 10 minutes.

### Example 11:

### Eye gel compositions

The rice culture from Example 1 was encapsulated into a liposomal composition, then the encapsulated extract was incorporated into an eye gel composition using the following process:

**EYE GEL containing Rice meristem conditioned nutrient media liposome**

| **Ingredient** | **INCI Nomenclature** | **%** |
|---|---|---|
| Water | Water | Q.S |
| Carbopol Ultrez 21 | Acrylates/C10-30 Alkyl Acrylate Crosspolymer | 0.50 |
| Keltrol CG-SFT | Xanthan Gum | 0.10 |
| Butylene Glycol | Butylene Glycol | 5.00 |
| Mikrokill COS | Phenoxyethanol & Caprylyl Glycol & Chlorphenesin | 1.00 |
| Dow Corning 193 Surfactant | Dimethicone Copolyol | 0.30 |
| Disodium EDTA | Disodium EDTA | 0.10 |
| AMP 95 | Aminomethylpropanol | 0.45 |
| Liposome containing rice culture from Example 1 | - | 1.00 |

### Procedure:

1. Disperse the Carbopol Ultrez 21 in water at 50 °C and add the Keltrol CG-SFT. Mix until uniform.
2. Add the Butylene Glycol, Mikrokill COS, AMP, EDTA and Silicone 193. Mix until uniform.
3. Add the Rice meristem conditioned nutrient media liposome with sweep agitation at 40 °C. Mix until uniform.
5. Adjust pH to 5.5 if necessary.

### Example 12:

### Encapsulated Extract from Example 1

The rice culture from Example 1 was encapsulated into a polymeric matrix using the techniques outlined in US Patent Application Publication No. 2003/0198682 A1.

### Example 13:

### Lipstick compositions

The rice culture from Example 1 was encapsulated in the polymeric matrix of Example 12, which was then formulated into a lipstick using the following formulation and process:

**LIPSTICK containing rice meristem conditioned nutrient medium**

| **Ingredient** | **INCI Nomenclature** | **%** |
|---|---|---|
| *Phase (A)* | | |
| Castor Oil | Ricinus Communis (Castor) Seed Oil | 31.45 |
| Schercemol TISC | Triisostearyl Citrate | 15.00 |
| Liquiwax PolyIPL | Stearyl PPG-3 Myristyl Ether Dimer Dilinoleate | 5.00 |
| Liquiwax PolyEFA | Octyldodecyl PPG-3 Myristyl Ether Dimer Dilinoloeate | 15.00 |
| Candelilla Wax | Euphorbia Cerifer (Candelilla) Wax | 6.00 |
| Ozokerite 170D | Ozokerite | 2.50 |
| Microwax SP 19 | Microcrystalline Wax | 3.50 |
| Carnauba Wax | Copernicia cerifera (carnauba) wax | 1.50 |
| Methylparaben | Methylparaben | 0.20 |
| Propylparaben | Propylparaben | 0.10 |

| *Phase (B)* | | |
|---|---|---|
| Color Grind | | |
| Red 7 Lake c19-7711 | Red 7 Lake | 0.04 |
| Red 6 Lake c 19-7712 | Red 6 Lake | 0.17 |
| Red Iron Oxide A-1205 | Iron Oxides | 2.00 |
| Titanium Dioxide Ultra Fine 70110 | Titanium Dioxide | 2.00 |
| Black Iron Oxide c33-134 | Iron Oxides | 0.05 |
| Liquiwax PolyEFA* | Octyldodecyl PPG-3 Myristyl Ether Dimer Dilinoloeate | 4.44 |

| *Phase (C)* | | |
|---|---|---|
| Ascorbyl Palmitate | Ascorbyl Palmitate | 0.05 |
| Flamenco Red | Mica and Titanium Dioxide | 10.00 |
| Powdered rice culture from Example 12 | - | 1.00 |

### Procedure:

Combine Waxes, Oils and Preservatives (Phase A) and heat to 83-87 °C.
Hold temperature and stir until homogeneous.
Drop temperature to 75-80 °C, and add Phase B; mix until homogeneous
Add Pearl, Rice meristem conditioned nutrient media and Ascorbyl Palmitate (Phase C).

### Pour into molds.

### Example 14:

### Toner compositions

The rice culture of Example 1 was formulated into an aqueous alcoholic tonic using the following formulation and process:

**Toner containing Rice meristem conditioned nutrient media**

| **Ingredient** | **INCI Nomenclature** | **%** |
|---|---|---|
| Water | Water | Qs. to 100 |
| Betafin BP-20* | Betaine | 3.00 |
| Rice Culture from Example 1 | - | 1.00 |
| Witch Hazel w/14 % Alcohol | Water & Ethanol & Witch Hazel | 25.00 |
| Mikrokill COS | Phenoxyethanol & Caprylyl Glycol & Chlorphenesin | 0.75 |

### Procedure:

1. Charge Water and add Betafin BP-20, and Rice meristem conditioned nutrient media. Mix until uniform.
2. Add Witch Hazel and Mikrokill COS, mix until uniform.

### Example 15:

### Body wash compositions

The rice culture of Example 1 was formulated into a body wash using the following formulation and process.

**Body Wash containing Rice meristem conditioned nutrient media**

| **Ingredient** | **INCI Nomenclature** | **%** |
|---|---|---|
| Water | Water | Q.S |
| Hamp-ene Na2 | Disodium EDTA | 0.10 |
| Glycerin | Glycerin | 2.00 |
| Standapol WAQ-Special | Sodium Lauryl Sulfate | 30.00 |
| Standapol ES-2 | Sodium Laureth Sulfate | 25.00 |
| Cerasynt IP | Glycol Stearate & Stearic Acid & Aminomethyl Propanol | 0.50 |
| Velvetex BA-35 | Cocoamidopropyl Betaine | 7.00 |
| Cocamide MEA | Cocamide MEA | 2.00 |
| Mikrokill COS | Phenoxyethanol & Caprylyl Glycol & Chlorphenesin | 0.75 |
| Rice Culture of Example 1 | | 1.00 |

### Procedure:

1. Heat Water to 70 °C and add Disodium EDTA, Glycerin, and mix until uniform.
2. Keep temperature above 70 °C and add Standapol WAQ Special, Standapol ES-2, Cerasynt IP, Cocamide MEA, Velvetex BA-35, and mix until uniform.
3. Cool to 45 °C and add Mikrokill COS and Osage orange extract.
4. Mix until homogenous.

### Example 16:

### Yeast/Example 1 Extract ferment products

The rice culture from Example 1 was included as part of a fermentation media containing the Yeast *Saccharomyces cerevisiae.* A sample of the extract from Example 1 was placed into an aqueous mixture of Baker's Yeast growth media obtained from Red Star Yeast (Milwaukee, WI). The media was inoculated with an active *Saccharomyces cerevisiae* yeast culture also obtained from Red Star and the mixture was allowed to ferment under controlled aerobic conditions to provide a Live Yeast Cell Derivative (LYCD) obtained using stress conditions as described in US patent 2,239,345.

### Example 17:

### Sub-micron emulsion concentrates

This example illustrates a sub-micron emulsion concentrate that contains rice culture prepared as described in Example 1.

| **Ingredient** | **Wt%** |
|---|---|
| Trimethylolpropane tricaprylate/tricaprate | 18.0 |
| Glycerin | 8.0 |
| Cetearyl alcohol | 2.0 |
| Ceteareth 20 | 2.0 |
| Glyceryl stearate | 2.0 |
| BHT | 0.01 |
| Rice culture from Example 1 | 1.0 |
| Water | to 100.0 |

### References

Ball MP, Li JB, Gao Y, Lee JH, LeProust EM, Park IH, Xie B, Daley GQ, Church GM (2009) Targeted and genome-scale strategies reveal gene-body methylation signatures in human cells. Nat Biotechnol 27(4): 361-368.
Bird A, Taggart M, Frommer M, Miller OJ, Macleod D (1985) A fraction of the mouse genome that is derived from islands of nonmethylated, CpG-rich DNA. Cell 40: 91-99.
Bird A (2002) DNA methylation patterns and epigenetic memory. Genes Dev 16: 6-21.
Boks MP, Derks EM, Weisenberger DJ, Strengman E, Janson E, Sommer IE, Kahn RS, Ophoff RA. (2009) The relationship of DNA methylation with age, gender and genotype in twins and healthy controls. PLoS One. 4(8): e6767.
Cropley JE, Suter CM, Beckman KB, Martin DI (2006) Germ-line epigenetic modification of the murine A vy allele by nutritional supplementation. Proc Natl Acad Sci 103: 17308-17312. Deng J, Shoemaker R, Xie B, Gore A, LeProust EM, Antosiewicz-Bourget J, Egli D, Maherali N, Park IH, Yu J, et al. (2009) Targeted bisulfate sequencing reveals changes in DNA methylation associated with nuclear reprogramming. Nat Biotechnol 27: 353-360.
Feng YQ, Desprat R, Fu H, Olivier E, Lin CM, et al. (2006) DNA methylation supports intrinsic epigenetic memory in mammalian cells. PLoS Genet 2: e65.
Fraga MF, Ballestar E, Paz MF, Ropero S, Setien F, et al. (2005) Epigenetic differences arise during the lifetime of monozygotic twins. Proc Natl Acad Sci 102: 10604-10609.
Gopisetty G, Ramachandran K, Singal R (2006) DNA methylation and apoptosis. Mol Immunol 43: 1729-1740.
Herskind AM, McGue M, Holm NV, Sorensen TI, Harvald B, Vaupel JW (1996) The heritability of human longevity: a population-based study of 2872 Danish twin pairs born 1870-1900. Hum Genet 97: 319-323.
Metivier R, Gallais R, Tiffoche C, Le PC, Jurkowska RZ, et al. (2008) Cyclical DNA methylation of a transcriptionally active promoter. Nature 452: 45-50.
Mitchell BD, Hsueh WC, King TM, Pollin TI, Sorkin J, Agarwala R, Schaffer AA, Shuldiner AR (2001) Heritability of life span in the old order Amish. Am J Med Genet 102: 346-352.
Ottaviano YL, Issa JP, Parl FF, Smith HS, Baylin SB, et al. (1994) Methylation of the estrogen receptor gene CpG island marks loss of estrogen receptor expression in human breast cancer cells. Cancer Res 54: 2552-2555.
Poulsen P, Esteller M, Vaag A, Fraga MF (2007) The epigenetic basis of twin discordance in age-related diseases. Pediatr Res 61: 38R-42R.
Rakyan VK, Chong S, Champ ME, Cuthbert PC, Morgan HD, et al. (2003) Transgenerational inheritance of epigenetic states at the murine Axin(Fu) allele occurs after maternal and paternal transmission. Proc Natl Acad Sci USA 100: 2538-2543.
Saxonov S, Berg P, Brutlag DL (2006) A genome-wide analysis of CpG dinucleotides in the human genome distinguishes two distinct classes of promoters. Proc Natl Acad Sci USA 103: 1412-1417.
Takatsu M, Uyeno S, Komura J, Watanabe M, Ono T (1999) Age-dependent alterations in mRNA level and promoter methylation of collagen alpha1(I) gene in human periodontal ligament. Mech Ageing Dev 110: 37-48.
Takai D, Jones PA (2002) Comprehensive analysis of CpG islands in human chromosomes 21 and 22. Proc Natl Acad Sci USA 99(6): 3740-3745.
Weaver IC, Meaney MJ, Szyf M (2006) Maternal care effects on the hippocampal transcriptome and anxiety-mediated behaviors in the offspring that are reversible in adulthood. Proc Natl Acad Sci USA 103: 3480-3485.

## Claims

1. A topical composition for controlling DNA methylation in human skin cells comprising:
(a) a plant meristem conditioned nutrient medium which is a plant meristem cell suspension extract and is derived from plants of the genus *Oryza*,
(b) a preservative present in an amount sufficient to provide sterilizing or biostatic efficacy with respect to component (a); and
(c) a dermatologically acceptable vehicle.

2. The composition of claim 1 wherein component (a) is present in an amount of 0.0000001 % to 99% by weight of the composition, with 0.0001 % to 50% being preferred, and 0.001% to 25% being more preferred and 0.01% to 10% being most preferred, component (b) is present in an amount of 0.01% to 10% by weight of the composition, with 0.1% to 5% being more preferred and 0.4% to 2% being most preferred, and component (c) is present in an amount of 1% to 98% by weight of the composition, with 80% to 90% being most preferred.

3. The composition of claim 1 or 2 wherein component (a) is derived from plants of *Oryza sativa.*

4. The composition of any of claims 1 to 3 wherein component (a) has been prepared by (i) growing plant meristems derived from plants of the genus *Oryza* in a liquid nutrient medium to produce a mixture containing water-soluble and water-insoluble components, and (ii) removing the water insoluble components, thereby producing the plant meristem conditioned nutrient medium.

5. The composition of any of claims 1 to 4 wherein the plant meristem cells have been grown in a liquid nutrient medium in the presence of chemical, gaseous, or energetic stresses intended to elicit expression of secondary plant cell metabolites.

6. The composition of claim 5 wherein the plant meristem cells have been grown in a liquid nutrient medium in the presence of ozone.

7. The composition of any of claims 1 to 6 wherein the preservative is selected from the group consisting of salicylic acid, sorbic acid, phenoxyethanol, benzyl alcohol, ethanol, Quaternium-15, glucose oxidase and lactoperoxidase in combination with a substrate, and combinations thereof.

8. The composition of claim 7 wherein the preservative is phenoxyethanol.

9. The composition of any of claims 1 to 8 wherein the preservative is present in an amount of at least 0.5 wt% based on the total weight of the composition.

10. The composition of any of claims 1 to 9 further comprising at least one ingredient selected from the group comprising water, surfactants, emulsifiers, conditioners, emollients, waxes, oils, polymers, thickeners, fixatives, colorants, humectants, moisturizers, stabilizers, diluents, solvents, fragrances, botanicals, nutraceuticals, cosmeceuticals, therapeutics, pharmaceuticals, antifungals, antimicrobials, steroidal hormones, antidandruff agents, anti-acne components, sunscreens, preservatives, and combinations thereof.

11. A composition concentrate comprising:
(a) a plant meristem conditioned nutrient medium which is a plant meristem cell suspension extract and is derived from plants of the genus *Oryza*, and
(b) a preservative present in an amount sufficient to provide sterilizing and biostatic efficacy with respect to component (a),
wherein component (a) is present in an amount of 0.0000001% to 99% by weight of the composition, with 90% to 99% being preferred, and 97% to 99% being more preferred, and component (b) is present in an amount of 0.01% to 10% by weight of the composition, with 0.1% to 5% being more preferred and 0.4% to 2% being most preferred.

12. The composition concentrate of claim 11 wherein component (a) is derived from plants of *Oryza sativa.*

13. A process for preparing the composition of claim 1 comprising the steps of:
(i) providing totipotent, undifferentiated plant cells derived from plants of the genus *Oryza*;
(ii) growing the plant cells in a liquid nutrient medium either in the presence of or the absence of chemical, gaseous, or energetic stresses intended to elicit expression of secondary plant cell metabolites to produce a mixture containing water-soluble and water-insoluble components,
(iii) removing the water insoluble components to produce the plant meristem conditioned nutrient medium which is a plant meristem cell suspension extract, and
(iv) combining the plant meristem conditioned nutrient medium with a preservative and a dermatologically acceptable vehicle thereby making the composition of claim 1.

14. The process of claim 13 wherein the plant cells of step (i) are derived from plants of *Oryza sativa.*

15. The process of claim 13 or 14 wherein the plant cells grow in the presence of ozone at step (ii).

16. The process of any of claims 13 to 15 wherein the plant cells of step (i) are prepared by a method comprising:
(1) growing plant meristems derived from plants of the genus *Oryza* in a solid nutrient medium; and
(2) selecting stabilized cultures thereby producing said plant cells.

## Patentansprüche

1. Topische Zusammensetzung zur Beeinflussung der DNS-Methylierung in menschlichen Hautzellen, umfassend:
(a) ein aufbereitetes Pflanzenmeristem-Nährmedium, welches ein Extrakt einer Suspension von pflanzlichen Meristemzellen ist und von Pflanzen der Gattung *Oryza* stammt,
(b) ein Konservierungsmittel in einer Menge, die ausreicht, um auf die Komponente (a) eine sterilisierende oder biostatische Wirkung auszuüben, und
(c) einen dermatologisch verträglichen Träger.

2. Zusammensetzung nach Anspruch 1, worin die Komponente (a) in einer Menge von 0,0000001% bis 99% des Gewichts der Zusammensetzung, vorzugsweise 0,0001% bis 50%, besonders bevorzugt 0,001% bis 25% und ganz besonders bevorzugt 0,01% bis 10% vorhanden ist, die Komponente (b) in einer Menge von 0,01% bis 10% des Gewichts der Zusammensetzung vorhanden ist, wobei 0,1% bis 5% besonders bevorzugt und 0,4% bis 2% am meisten bevorzugt sind, und Komponente (c) in einer Menge von 1% bis 98% des Gewichts der Zusammensetzung anwesend ist, wobei 80% bis 90% ganz besonders bevorzugt sind.

3. Zusammensetzung nach Anspruch 1 oder 2, worin die Komponente (a) von Pflanzen von *Oryza sativa* stammt.

4. Zusammensetzung nach einem der Ansprüche 1 bis 3, worin die Komponente (a) hergestellt wurde durch (i) Kultivieren von Pflanzenmeristemen, die von Pflanzen der Gattung *Oryza* stammen, in einem flüssigen Nährmedium zur Gewinnung einer wasserlösliche und wasserunlösliche Komponenten enthaltenden Mischung, und (ii) Entfernung der wasserunlöslichen Komponenten zur Gewinnung des aufbereiteten Pflanzenmeristem-Nährmediums.

5. Zusammensetzung nach einem der Ansprüche 1 bis 4, worin die pflanzlichen Meristemzellen in einem flüssigen Nährmedium in Gegenwart von chemischen, gasförmigen oder energetischen Belastungen kultiviert wurden, um die Expression von sekundären Pflanzenzell-Metaboliten auszulösen.

6. Zusammensetzung nach Anspruch 5, worin die pflanzlichen Meristemzellen in einem flüssigen Nährmedium in Gegenwart von Ozon kultiviert wurden.

7. Zusammensetzung nach einem der Ansprüche 1 bis 6, worin das Konservierungsmittel aus der aus Salicylsäure, Sorbinsäure, Phenoxyethanol, Benzylalkohol, Ethanol, Quaternium-15, Glucoseoxidase und Lactoperoxidase in Kombination mit einem Substrat, und Kombinationen der Genannten bestehenden Gruppe ausgewählt ist.

8. Zusammensetzung nach Anspruch 7, worin das Konservierungsmittel Phenoxyethanol ist.

9. Zusammensetzung nach einem der Ansprüche 1 bis 8, worin das Konservierungsmittel in einer Menge von wenigstens 0,5%, bezogen auf das Gesamtgewicht der Zusammensetzung, anwesend ist.

10. Zusammensetzung nach einem der Ansprüche 1 bis 9, welche zusätzlich wenigstens einen aus der aus Wasser, Tensiden, Emulgatoren, Konditionierern, Weichmachern, Wachsen, Ölen, Polymeren, Verdickungsmitteln, Fixativen, Färbemitteln, Feuchthaltemitteln, Feuchtmachern, Stabilisatoren, Verdünnungsmitteln, Lösemitteln, Duftstoffen, Pflanzenextrakten, Nutrazeutika, Kosmezeutika, Therapeutika, Arzneimitteln, Antimykotika, antimikrobiellen Substanzen, Steroidhormonen, Antischuppenmitteln, Antiaknewirkstoffen, Sonnenschutzmitteln, Konservierungsmitteln und Kombinationen der Genannten bestehenden Gruppe ausgewählten Bestandteil umfasst.

11. Zusammensetzungskonzentrat, welches
(a) ein aufbereitetes Pflanzenmeristem-Nährmedium, welches ein Extrakt einer Suspension von pflanzlichen Meristemzellen ist und von Pflanzen der Gattung *Oryza* stammt, und
(b) ein Konservierungsmittel in einer Menge, die ausreicht, um auf die Komponente (a) eine sterilisierende oder biostatische Wirkung auszuüben,
umfasst und worin Komponente (a) in einer Menge von 0,0000001% bis 99% des Gewichts der Zusammensetzung vorhanden ist, wobei 90% bis 99% bevorzugt und 97% bis 99% besonders bevorzugt sind, und Komponente (b) in einer Menge von 0,01% bis 10% des Gewichts der Zusammensetzung vorhanden ist, wobei 0,1% bis 5% bevorzugt und 0,4% bis 2% besonders bevorzugt sind.

12. Zusammensetzungskonzentrat nach Anspruch 11, worin die Komponente (a) von Pflanzen von *Oryza sativa* stammt.

13. Verfahren zur Herstellung der Zusammensetzung gemäß Anspruch 1, umfassend die Schritte
(i) Bereitstellen von totipotenten, undifferenzierten, von Pflanzen der Gattung *Oryza* stammenden Pflanzenzellen,
(ii) Kultivieren der Pflanzenzellen in einem flüssigen Nährmedium, entweder in Gegenwart oder in Abwesenheit von chemischen, gasförmigen oder energetischen Belastungen, die dazu bestimmt sind, die Expression von sekundären Pflanzenzell-Metaboliten auszulösen, zur Gewinnung einer wasserlösliche und wasserunlösliche Komponenten enthaltenden Mischung,
(iii) Entfernung der wasserunlöslichen Komponenten zur Gewinnung des aufbereiteten Pflanzenmeristem-Nährmediums, welches ein Extrakt einer Suspension von pflanzlichen Meristemzellen ist, und
(iv) Vermischung des aufbereiteten Pflanzenmeristem-Nährmediums mit einem Konservierungsmittel und einem dermatologisch verträglichen Träger, wodurch die Zusammensetzung gemäß Anspruch 1 erhalten wird.

14. Verfahren nach Anspruch 13, worin die Pflanzenzellen in Schritt (i) von Pflanzen von *Oryza sativa* stammen.

15. Verfahren nach Anspruch 13 oder 14, worin die Pflanzenzellen in Schritt (ii) in Gegenwart von Ozon wachsen.

16. Verfahren nach einem der Ansprüche 13 bis 15, worin die Pflanzenzellen von Schritt (i) durch ein Verfahren erhalten werden, welches
(1) das Kultivieren von Pflanzenmeristemen, die von Pflanzen der Gattung *Oryza* stammen, in einem festen Nährmedium, und
(2) die Selektion stabiler Kulturen zur Gewinnung besagter Pflanzenzellen umfasst.

## Revendications

1. Composition topique pour contrôler la méthylation d'ADN dans des cellules cutanées humaines comprenant :
(a) un milieu nutritif conditionné de méristème de plante qui est un extrait de suspension de cellules de méristème de plante et est dérivé de plantes du genre *Oryza*,
(b) un conservateur présent en une quantité suffisante pour obtenir une efficacité de stérilisation ou biostatique vis-à-vis du composant (a) ; et
(c) un véhicule acceptable en dermatologie.

2. Composition de la revendication 1 dans laquelle le composant (a) est présent en une quantité de 0,0000001 % à 99 % en poids de la composition, 0,0001 % à 50 % étant préféré, et 0,001 % à 25 % étant plus préféré et 0,01 % à 10 % étant préféré entre tous, le composant (b) est présent en une quantité de 0,01 % à 10 % en poids de la composition, 0,1 % à 5 % étant plus préféré et 0,4 % à 2 % étant préféré entre tous, et le composant (c) est présent en une quantité de 1 % à 98 % en poids de la composition, 80 % à 90 % étant préféré entre tous.

3. Composition de la revendication 1 ou 2 dans laquelle le composant (a) est dérivé de plantes d'*Oryza sativa.*

4. Composition de l'une quelconque des revendications 1 à 3 dans laquelle le composant (a) a été préparé par (i) culture de méristèmes de plante dérivés de plantes du genre *Oryza* dans un milieu nutritif liquide pour produire un mélange contenant des composants solubles dans l'eau et insolubles dans l'eau, et (ii) élimination des composants insolubles dans l'eau, de manière à produire le milieu nutritif conditionné de méristème de plante.

5. Composition de l'une quelconque des revendications 1 à 4 dans laquelle les cellules de méristème de plante ont été cultivées dans un milieu nutritif liquide en présence de stress chimiques, gazeux ou énergétiques destinés à induire l'expression de métabolites de cellules de plante secondaires.

6. Composition de la revendication 5 dans laquelle les cellules de méristème de plante ont été cultivées dans un milieu nutritif liquide en présence d'ozone.

7. Composition de l'une quelconque des revendications 1 à 6 dans laquelle le conservateur est choisi dans le groupe constitué de l'acide salicylique, l'acide sorbique, le phénoxyéthanol, l'alcool benzylique, l'éthanol, Quaternium-15, la glucose oxydase et la lactoperoxydase en combinaison avec un substrat, et des combinaisons de ceux-ci.

8. Composition de la revendication 7 dans laquelle le conservateur est le phénoxyéthanol.

9. Composition de l'une quelconque des revendications 1 à 8 dans laquelle le conservateur est présent en une quantité d'au moins 0,5 % en poids sur la base du poids total de la composition.

10. Composition de l'une quelconque des revendications 1 à 9 comprenant en outre au moins un composant choisi dans le groupe comprenant l'eau, des tensioactifs, des émulsifiants, des conditionneurs, des émollients, des cires, des huiles, des polymères, des épaississants, des fixateurs, des colorants, des humectants, des hydratants, des stabilisants, des diluants, des solvants, des parfums, des substances botaniques, nutraceutiques, cosméceutiques, thérapeutiques, pharmaceutiques, antifungiques, antimicrobiennes, des hormones stéroïdiennes, des agents antipelliculaires, des composants anti-acné, des écrans solaires, des conservateurs, et des combinaisons de ceux-ci.

11. Composition concentrée comprenant :
(a) un milieu nutritif conditionné de méristème de plante qui est un extrait de suspension de cellules de méristème de plante et est dérivé de plantes du genre *Oryza,* et
(b) un conservateur présent en une quantité suffisante pour obtenir une efficacité de stérilisation ou biostatique vis-à-vis du composant (a),
dans laquelle le composant (a) est présent en une quantité de 0,0000001 % à 99 % en poids de la composition, 90 % à 99 % étant préféré, et 97 % à 99 % étant plus préféré, et le composant (b) est présent en une quantité de 0,01 % à 10 % en poids de la composition, 0,1 % à 5 % étant plus préféré et 0,4 % à 2 % étant préféré entre tous.

12. Composition concentrée de la revendication 11 dans laquelle le composant (a) est dérivé de plantes d'*Oryza sativa.*

13. Procédé de préparation de la composition de la revendication 1 comprenant les étapes de :
(i) fourniture de cellules de plante totipotentes, indifférenciées dérivées de plantes du genre *Oryza* ;
(ii) culture des cellules de plante dans un milieu nutritif liquide en présence ou en l'absence de stress chimiques, gazeux ou énergétiques destinés à induire l'expression de métabolites de cellules de plante secondaires pour produire un mélange contenant des composants solubles dans l'eau et insolubles dans l'eau,
(iii) élimination des composants insolubles dans l'eau pour produire le milieu nutritif conditionné de méristème de plante qui est un extrait de suspension de cellules de méristème de plante, et
(iv) combinaison du milieu nutritif conditionné de méristème de plante avec un conservateur et un véhicule acceptable en dermatologie de manière à produire la composition de la revendication 1.

14. Procédé de la revendication 13 dans lequel les cellules de plante de l'étape (i) sont dérivées de plantes d'*Oryza sativa.*

15. Procédé de la revendication 13 ou 14 dans lequel les cellules de plante croissent en présence d'ozone à l'étape (ii).

16. Procédé de l'une quelconque des revendications 13 à 15 dans lequel les cellules de plante de l'étape (i) sont préparées par un procédé comprenant :
(1) la culture de méristèmes de plante dérivés de plantes du genre *Oryza* dans un milieu nutritif solide ; et
(2) la sélection de cultures stabilisées de manière à produire lesdites cellules de plante.
